(19) Europäisches Patentamt
European Patent Office
Office européen des brevets

(11) **EP 2 293 035 B1**

(12) **EUROPÄISCHE PATENTSCHRIFT**

(45) Veröffentlichungstag und Bekanntmachung des Hinweises auf die Patenterteilung:
**22.12.2021   Patentblatt 2021/51**

(51) Int Cl.:
***G01J 9/00*** *(2006.01)*      ***A61B 3/103*** *(2006.01)*

(21) Anmeldenummer: **10006444.3**

(22) Anmeldetag: **22.06.2010**

(54) **VERFAHREN UND VORRICHTUNG ZUR DARSTELLUNG EINER ABTASTFUNKTION**

METHOD AND DEVICE FOR DISPLAYING A SAMPLING FUNCTION

PROCEDE ET DISPOSITIF D'AFFICHAGE D'UNE FONCTION DE BALAYAGE

(84) Benannte Vertragsstaaten:
**DE FR GB IT PL**

(30) Priorität: **24.06.2009   DE 102009027165**

(43) Veröffentlichungstag der Anmeldung:
**09.03.2011   Patentblatt 2011/10**

(73) Patentinhaber: **Institut Franco-Allemand de Recherches de Saint-Louis**
**68301 Saint-Louis Cedex (FR)**

(72) Erfinder:
 • **Raymond, Pierre F.**
   **68990 Heimsbrunn (FR)**
 • **Eichhorn, Marc D.**
   **79736 Rickenbach (DE)**
 • **Pichler, Alexander D.**
   **68130 Altkirch (FR)**

(56) Entgegenhaltungen:
 • **GUO, H. ET AL.: "Wavefront reconstruction with artificial neural networks", OPTICS EXPRESS, Bd. 14, Nr. 14, 10. Juni 2006 (2006-06-10) , Seiten 6456-6462, XP002606609,**
 • **General Vision, Data Sheet: "V1KU Image recognition module", , März 2009 (2009-03), XP002606610, Gefunden im Internet: URL:http://www.general-vision.com/Datasheet/DS_V1KU.pdf [gefunden am 2010-10-22]**
 • **QI B. ET AL.: "Wavefront fitting of interferograms with Zernike polynomials", OPT. ENG., Bd. 1, Juli 2002 (2002-07), Seiten 1565-1569, XP002606611,**
 • **None**

**Beschreibung**

Die vorliegende Erfindung betrifft ein Verfahren zur Darstellung einer Abtastfunktion mittels eines neuronalen Netzwerkes gemäß dem Anspruch 1 sowie eine Vorrichtung zur Durchführung des Verfahrens gemäß dem Anspruch 8.

Eine Veröffentlichung (GUO, H. ET AL.:"Wavefront reconstruction with artificial neural networks", OPTICS EXPRESS, Bd. 14, Nr. 14, 10. Juni 2006 (2006-06-10), Seiten 6456-6462) zeigt ein Verfahren mit den Merkmalen des Oberbegriffes des Patentanspruches 1 und eine Vorrichtung mit den Merkmalen des Oberbegriffes des Anspruches 8. Das neuronale Netzwerk berechnet Werte von Koeffizienten einer Repräsentationsfunktion in einem einzigen Schritt.

Eine Veröffentlichung (General Vision, Data Sheet: "VIKU Image recognition module", März 2009 (2009-03)) zeigt ein Verfahren zur Darstellung einer Abtastfunktion mittels eines neuronalen Netzwerks, mit folgenden Schritten:

- die Abtastfunktion wird dem neuronalen Netzwerk zur Verarbeitung übermittelt;
- eine Anzahl von Vergleichsfunktionen wird dem neuronalen Netzwerk zur Verfügung gestellt;
- durch Vergleich der Abtastfunktion mit mehreren der Vergleichsfunktionen wird eine primäre Vergleichsfunktion selektiert;
- die selektierte primäre Vergleichsfunktion wird als dominante Repräsentationsfunktion bestimmt.

Wird ein Abtaststrahl ausgesandt und dessen Respons analysiert, kommt es auf diverse störende Einflüsse an, die eine Analyse und Auswertung der Respons erschweren oder sogar unmöglich machen können. Insbesondere bei der Verwendung der Lasertechnik spielt die Strahlqualität und die Fokussierbarkeit des Laserstrahls eine große Rolle. Auch eine hohe optische Auflösung der Respons eines Laserstrahls hat einen großen Einfluss auf das Ergebnis einer Abtastung.

Wird dementsprechend ein Laserstrahl ausgesandt und dessen Respons erfasst, trifft eine turbulente Wellenfront, die eine Abtastfunktion darstellen kann, auf eine bevorzugt wenigstens zweidimensionale Anordnung von optischen Elementen, etwa Linsen, Beugungsgitter oder dgl., die die einfallende Abtastfunktion in Phaseninformationen enthaltene Bestandteile zu wandeln vermag. Die Bestandteile der Abtastfunktion, die beispielsweise durch ein Mikrolinsen-Array zerlegt werden, treffen auf einen Messsensor mit einer zweidimensionalen Struktur von Messelementen. Als ein derartiger Sensor kann beispielsweise eine CCD- Sensormatrix angesehen werden.

Im Falle einer planen Wellenfront, d.h. einer Phasen- bzw. Wellenfront senkrecht zur Strahl-Ausbreitungsrichtung, würde das Mikrolinsen-Array die eintreffende Wellenfront derart zerlegen, dass Referenzpunkte auf der CCD-Matrix, die bevorzugt äquidistant sind, getroffen werden würden. Da aber die eintreffende Wellenfront als Respons eines kohärenten Laserstrahls eine turbulente Wellenfront mit beträchtlichen Phasenstörungen ist, werden die prinzipiell als Gitter anzusehenden Linsen des Mikrolinsen-Arrays Abbildungspunkte auf der CCD-Matrix erzeugen, die gegenüber den Referenzpunkten ausgelenkt sind.

Üblicherweise wird die Verschiebung der gemessenen Punkte gegenüber den Referenzpositionen, die ein Maß für die lokale Phasenfrontneigung ist, über eine Integration der realen Phasenfront bestimmt. Dabei ist nur noch ein absoluter Offset zu berücksichtigen. Ein weiteres Verfahren erzeugt ein über das gesamte Bild der CCD-Matrix verteiltes, der Phasenfunktion zugeordnetes Interferenzmuster, aus dem die lokale Phasenfrontneigung ebenfalls berechnet werden kann.

Die genannten Verfahren sind jedoch sehr zeitaufwändig, so dass eine Korrektur in Echtzeit oder in nahezu Echtzeit nur in den einfachsten Fällen ermöglicht werden kann. In realistischen Fällen, wie sie etwa bei Anwendungsfällen in der Augenheilkunde, der Mustererkennung bei der Navigation oder auf dem militärischen Gebiet nahezu regelmäßig vorkommen, ist eine derartige Korrektur nicht möglich. Dort müssen Bilder zwischengespeichert werden und die Auswertung der Bilder muss nachträglich erfolgen.

Es ist dementsprechend eine Aufgabe der vorliegenden Erfindung, wenigstens einigen der Nachteile des Standes der Technik wenigstens teilweise Abhilfe zu verschaffen. Insbesondere soll es ermöglicht werden, die besagten Phasenkorrekturen auch in komplexen Fällen nahezu in Echtzeit vorzunehmen.

Die gemäß der Erfindung erzielbaren Vorteile beruhen auf einem Verfahren und einer Vorrichtung gemäß dem jeweiligen unabhängigen Anspruch. Zweckmäßige Ausführungsformen der erfindungsgemäßen Gegenstände werden in den Unteransprüchen definiert.

Zur Darstellung einer Abtastfunktion mittels eines neuronalen Netzwerkes wird zunächst die Abtastfunktion optisch in Phaseninformationen enthaltende Teile zerlegt. Nachfolgend werden die Phaseninformationen enthaltenden Bestandteile dem neuronalen Netzwerk zur Verarbeitung übermittelt. Das neuronale Netzwerk hat Zugriff auf eine Anzahl von Vergleichsfunktionen bzw. ist das neuronale Netzwerk auf diese Vergleichsfunktionen angelernt. Durch Vergleich der Phaseninformationen mit einer oder mehreren der Vergleichsfunktionen wird eine primäre Vergleichsfunktion selektiert, wobei als Kriterium für die Selektion eine vorgebbare Abweichung oder ein Abweichungsextremum dient. Die selektierte primäre Vergleichsfunktion wird als Repräsentationsfunktion und bevorzugt als dominante Repräsentationsfunktion bestimmt.

Eine Vorrichtung, die auch zur Durchführung des erfindungsgemäßen Verfahrens einsetzbar ist, umfasst eine wenigstens zweidimensionale Empfängeranordnung mit einer Anzahl von darauf verteilten, vorzugsweise optischen Sensoren bzw. Detektoren. Eine wenigstens zweidimensionale Anordnung von optischen Elementen, etwa Linsen, Beugungsgitter oder dgl., die eine einfallende Abtastfunktion in Phaseninformationen enthaltene Bestandteile wandelt, ist vor der Empfängeranordnung, etwa einer CCD-Sensormatrix, vorgesehen. In das neuronale Netzwerk werden die Bestandteile, die phasenrelevante Informationen enthalten, die von der Empfängeranordnung kommen, eingegeben. Das neuronale Netzwerk vermag auf einen Speicher mit einer Anzahl von Vergleichsfunktionen zurückzugreifen, bzw. ist auf diese Vergleichsfunktionen angelernt. So vermag das neuronale Netzwerk die Bestandteile der Abtastfunktionen mit wenigstens einer der Vergleichsfunktionen zu vergleichen, wenigstens eine der Vergleichsfunktionen als vorzugsweise dominante Vergleichsfunktion zu selektieren und die Abtastfunktion damit zu beschreiben.

Dabei ist es durchaus wesentlich, dass die primären Vergleichsfunktionen einer vollständigen Funktionenbasis entstammen bzw. wenigstens eine Untermenge der Funktionen einer derartigen Funktionenbasis bilden. Diese Funktionen bilden einen Satz von Interferrometriemustern, die in einer turbulenten Wellenfront, die auszumessen ist, als Phasenverteilungsfunktionen wieder zu finden sind. Das neuronale Netzwerk erhält als Eingangsinformation die Bestandteile bzw. Eingangsvektoren der Verschiebungsvektoren aller Punkte der Abtastfunktionen und vermag diese parallel zu verarbeiten. Das Netzwerk vermag nun die Funktionen aus der vollständigen Funktionenbasis, auf die das neuronale Netzwerk angelernt ist bzw. auf die es zugreifen kann und deren Amplitude in der Abtastfunktion zu erkennen. Somit ist es möglich, die turbulente Wellenfront, die gemessen worden ist und als Abtastfunktion zugänglich ist, mit den erkannten Basisfunktionen einer vollständigen Funktionenbasis mathematisch zu rekonstruieren. Als Beispiel für ein verwendbares neuronale Netzwerk kann ein Netzwerk vom Typ "CogniMem" zum Einsatz gelangen, welches von der Firma General Vision, USA, bezogen werden kann.

Nachfolgend wird unter Verweis auf die beigefügten Figuren eine bevorzugte Ausführungsform gemäß der Erfindung näher erläutert. Dabei werden weitere erfindungsgemäße Merkmale und Vorzüge beschrieben. In den Figuren zeigen:

Fig. 1   eine Prinzipdarstellung einer Messanordnung, die gemäß der Erfindung einsetzbar ist;

Fig. 2   einige Funktionen, hier Zernike-Polynome, und diesen zugeordnete Abberationen;

Fig. 3   ein Beispiel eines erfassten Punktmusters einer Abtastfunktion relativ zu Referenzpunkten einer idealen Respons;

Fig. 4   eine Normalisierung von Verschiebungen von gemessenen Punkten gegenüber Referenzpunkten auf 8-Bit-Werte;

Fig. 5   eine Auswahl von Vergleichsfunktionen zum Vergleich einer Abtastfunktion mit den Vergleichsfunktionen aus einer neuronalen Musterdatenbank bzw. für ein neuronales Netzwerk; und

Fig. 6   ein Beispiel für ein prinzipielles Ablaufschema zur Darstellung einer Abtastfunktion mittels in der Abtastfunktion erkannten Vergleichsfunktionen.

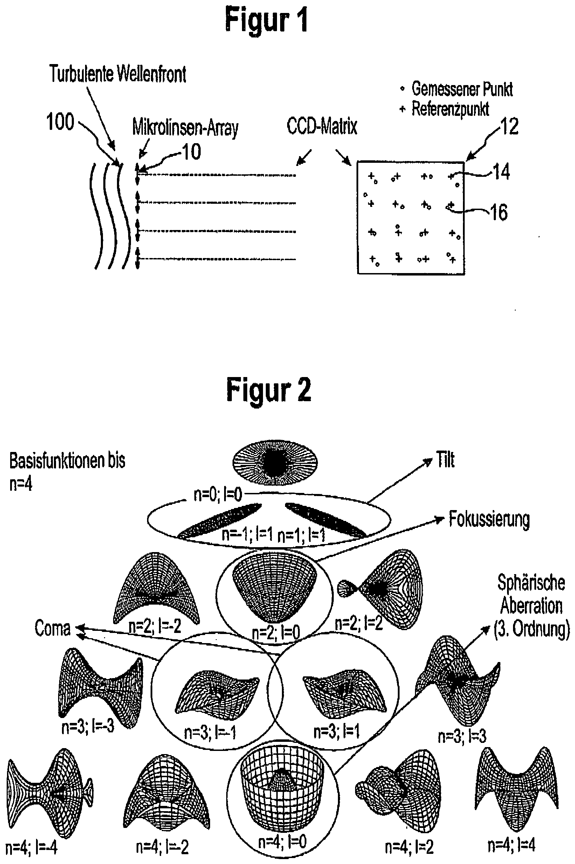

Der Figur 1 ist eine prinzipielle Anordnung eines Detektors zu entnehmen. Dargestellt ist ein Shack-Hartmann-Sensor (SHS), auf dem eine turbulente Wellenfront 100 auftrifft. Die Wellenfront trifft auf ein zweidimensionales Mikrolinsen-Array, das die turbulente Wellenfront in verschiedene Maxima zerlegt, die von den jeweiligen Linsen erzeugt werden. Eine nicht turbolente Wellenfront würde ein Muster auf einem zweidimensionalen Detektor, etwa einer CCD-Sensormatrix, entstehen lassen, die eine ideale Ausbildung hat, wobei äquidistante Abbildungen durch die verschiedenen Mikrolinsen auf der CCD-Sensormatrix entstehen würden.

Dementsprechend entstehen auf der CCD-Sensormatrix 12 im Idealfall Abbildungspunkte 14, insoweit die einfallende Wellenfront plan ist und senkrecht auf das Linsenarray einfällt. Insofern es sich um eine turbulente Wellenfront 100 handelt, werden die inkohärenten Bestandteile der Wellenfront zu einer Versetzung der Abbildungspunkte der Mikrolinsen des Mikrolinsen-Arrays 10 führen und damit zu Abbildungspunkten 16 auf der CCD-Sensormatrix 12. Der Versatz zwischen den Abbildungspunkten 16 und den Referenzpunkten 14 entspricht der lokalen Phasenfrontneigung. Neben dem Shack-Hartmann-Verfahren kommt zu Zwecken der Erfindung beispielsweise auch die Vierwellen-Lateral-Shearing-Interferometrie in Betracht.

Die gemessene Phasenverteilung ist nachfolgend zu analysieren, um diese mathematisch in Vergleichsfunktionen einer vollständigen Funktionenbasis zu zerlegen.

In der Figur 2 sind beispielhaft einige Zernike-Polynome dargestellt, mit denen eine eindeutige Zerlegung einer gemessenen Phasenverteilung einer Wellenfront vorgenommen werden kann.

Gemäß der Erfindung wird einem neuronalen Netzwerk ein Satz von Vergleichsfunktionen zugänglich gemacht bzw. wird das neuronale Netzwerk auf einen Satz von Vergleichsfunktionen angelernt. Diese bilden eine Untermenge von Funktionen einer vollständigen Funktionenbasis und im Falle von Figur 2, der Zernike-Polynome. Dem neuronalen Netzwerk werden als Eingangssektoren Verschiebungsvektoren aller Punkte eingespeist. Aufgrund der Struktur neuronaler Netzwerke können diese Informationen parallel verarbeitet werden. Ein Vorteil der erfindungsgemäßen Vorgehensweise liegt darin, dass die Verwendung einer größeren Anzahl von Referenzpunkten bzw. gemessenen Punkten aufgrund des speziellen Netzwerkes nicht zu einer Ausdehnung der Verarbeitungszeit führt. Andererseits hat die Vergrößerung der Anzahl von Referenz- bzw. Messpunkten eine bessere Auflösung zur Folge.

Um die Erkennung von Vergleichsfunktionen innerhalb der Abtastfunktion durchführen zu können, ist es erforderlich, zunächst ein erfasstes Muster mit Messpunkten beispielsweise mittels Schwerpunktberechnung in Matrizen zu wandeln. Dabei werden zunächst zu der einer Linse des Mikrolinsen-Arrays zugeordneten Kamerazelle zwei Matrizen $X_C$ und $Y_C$ berechnet, die die X- und Y-Positionen der Punkte enthalten. In dem nachfolgend dargestellten Beispiel werden die Positionen auch in Gleichung (1) in Metern aufgeführt.

$$X_\mathrm{C} = \begin{bmatrix} X_{C1} & X_{C2} & \cdots & \cdots \\ \cdots & \cdots & \cdots & \cdots \\ \cdots & X_{Ci} & \cdots & \cdots \\ \cdots & \cdots & \cdots & X_{Cn} \end{bmatrix} \qquad Y_\mathrm{C} = \begin{bmatrix} Y_{C1} & Y_{C2} & \cdots & \cdots \\ \cdots & \cdots & \cdots & \cdots \\ \cdots & Y_{Ci} & \cdots & \cdots \\ \cdots & \cdots & \cdots & Y_{Cn} \end{bmatrix} \qquad (1)$$

Zwei Referenz-Matrizen $X_R$ und $Y_R$ gemäß Gleichung (2) beschreiben die Mittelpunkte der jeder Linse auf der CCD-Sensormatrix zugeordneten Positionen.

$$X_\mathrm{R} = \begin{bmatrix} X_{R1} & X_{R2} & \cdots & \cdots \\ \cdots & \cdots & \cdots & \cdots \\ \cdots & X_{Ri} & \cdots & \cdots \\ \cdots & \cdots & \cdots & X_{Rn} \end{bmatrix} \qquad Y_\mathrm{R} = \begin{bmatrix} Y_{R1} & Y_{R2} & \cdots & \cdots \\ \cdots & \cdots & \cdots & \cdots \\ \cdots & Y_{Ri} & \cdots & \cdots \\ \cdots & \cdots & \cdots & Y_{Rn} \end{bmatrix} \qquad (2)$$

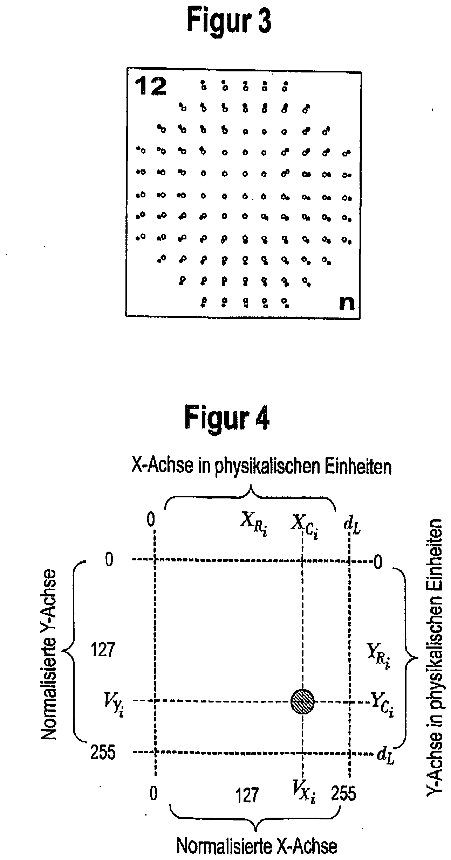

Die in Figur 3 gezeigten Punktmuster, einerseits die Referenzpunkte und andererseits die Punkte des Abtastmusters stellen ein Beispiel dar, wie es etwa auch aus Figur 1 hervorgeht. Dabei ergeben sich für die als Punkte dargestellten Musterbestandteile, die Messwerte darstellen, die Koordinaten $(X_{ci}, Y_{ci})$. Die als Kreuze sichtbar gemachten Punkte beziehen sich auf die Referenzwerte, auf die die Messwerte idealerweise direkt projiziert werden sollten, insofern die auftreffende Wellenfront nicht turbulent wäre. Diese Referenzwerte werden als $(X_{Ri}, Y_{Ri})$ im Folgenden bezeichnet, wobei i = 1 ... n läuft und n der Anzahl der Linsen des Arrays entspricht.

Das folgende Beispiel veranschaulicht den neuronalen Algorithmus anhand der aktuellen bzw. einsetzbaren CogniMem-Technologie, welche in der Lage ist, maximal 256 Bytes (8 Bit-Worte) lange Eingangsvektoren zu klassifizieren. Das Verfahren nach der Erfindung ist nicht auf diese Technologie beschränkt. Basierend auf den zuvor bestimmten Messungs- und Referenzmatrizen und unter Kenntnis der Linsen-Breite/Länge *dL* (in Metern) errechnet sich der dem Punktmuster entsprechende Eingangsvektor des neuronalen Netzwerks wie folgt (Gleichung (3), (3.1) und (3.2)):

$$V = \begin{pmatrix} V_{X1} & V_{Y2} & \cdots & \cdots & V_{Xn} & V_{Yn} \end{pmatrix} \qquad (3)$$

$$V_\mathrm{Xi} = \left[ \frac{\left( X_{Ci} - X_{Ri} + \dfrac{d_L}{2} \right)}{d_L} \cdot 255 \right] \qquad (3.1)$$

$$V_\mathrm{Yi} = \left[ \frac{\left( Y_{Ci} - Y_{Ri} + \dfrac{d_L}{2} \right)}{d_L} \cdot 255 \right] \qquad (3.2)$$

Somit werden alle Referenzpunkte $(X_{Ri}, YR_{Ri})$ auf den Punkt (127,127) abgebildet.

Die Messpunkte $(X_{Ci}, Y_{Ci})$ werden innerhalb der Linsen-Zelle auf den Bereich zwischen 0 und 255 abgebildet (siehe auch Figur 3). Die Länge des Eingangsvektors $L_v$ errechnet sich aus der Anzahl der Linsen $n_{Lx}$ und $n_{LY}$ (in X- bzw. Y-Richtung), die zur Abbildung der Phasenfront genutzt werden (Gleichung (4)).

$$L_v = 2 \cdot n_{Lx} \cdot n_{LY} \tag{4}$$

Die Maximallänge von 256 Bytes darf in diesem Beispiel, wie in Figur 4 erkennbar, nicht überschritten werden. Daher werden wie in Figur 4 gezeigt, hier nur 11 x 11 Linsen zur Rekonstruktion der Phasenfront genutzt. Demzufolge weist der entsprechende Eingangsvektor also eine Länge von 242 Bytes auf.

Die so konstruierten Vektoren können nun dem neuronalen Netzwerk sowohl als Muster zum Anlernen als auch zur Erkennung übergeben werden. In den nachfolgenden Gleichungen wird die Transformation von Phasenverteilungen in die zugehörigen Eingangsvektoren wie folgt beschrieben:

$$V = \psi\,(\varphi) \tag{5}$$

In Gleichung (5) steht $V$ für den resultierenden Eingangsvektor, der aus der ursprünglichen Phasenverteilung $\varphi$ durch Anwendung des Shack-Hartmann-Verfahrens, sowie Durchführung der in den Gleichungen (3), (3.1) und (3.2) beschriebenen Transformationen entsteht.

Die beschriebene Transformation ist dabei reversibel, d.h. unter Kenntnis der zur Transformation verwendeten physikalischen Daten (Längen, etc.) lässt sich aus einem bekannten Eingangsvektor $V$ jederzeit die zugehörige Phasenverteilung $\varphi$ wiederherstellen.

Diese Rücktransformation $\psi'$ wird in Gleichung (6) beschrieben:

$$\varphi = \psi'\,(V) \tag{6}$$

Um das neuronale Netzwerk zur Klassifizierung von Phasenfronten einsetzen zu können, ist es nötig, zum Zeitpunkt der Konzeption des Systems eine Datenbank mit bekannten Grundphasenverteilungen zu erzeugen, die speziellen optischen Störungen entsprechen, und das neuronale Netzwerk auf diese anzulernen.

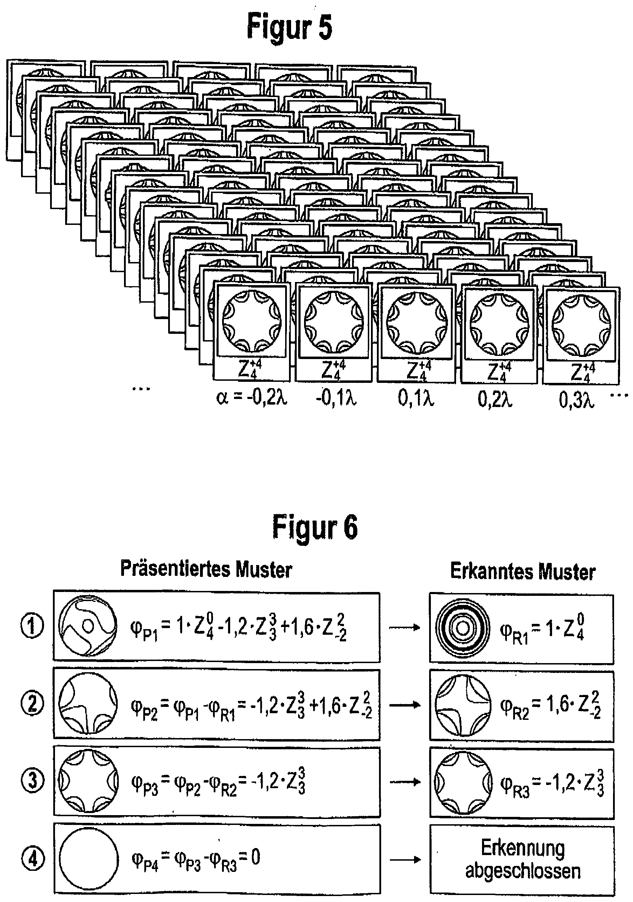

Hierfür bieten sich z.B. die bereits erwähnten Zemike-Polynome an, welchen eine direkte physikalische Bedeutung zugeordnet werden kann. Je nach Messaufgabe wird eine Auswahl von gewünschten Polynomen erstellt und ein Bereich sowie eine Schrittweite für die Diskretisierung der Amplitude der so beschriebenen Phasenstörung festgelegt. Die zugehörigen Phasenverteilungen können dann rechnerisch ermittelt und als Eingangsvektor umgewandelt dem neuronalen Netzwerk angelernt werden. In Figur 5 ist grafisch ein Beispiel eines Ausschnitts aus einer so erstellten neuronalen Datenbank dar, hier bestehend aus den 15 ersten Zernike-Polynomen (bis einschließlich der Ordnung 4) mit einer Amplitudendiskretisierung von $0{,}1\lambda$. Die Genauigkeit der vom neuronalen Netzwerk gelieferten Erkennungsergebnisse hängt ab vom festgelegten Bereich und der Diskretisierung der Amplitude, sowie von der Anzahl von gelernten Grund-Polynomen. Dies bedeutet natürlich, dass die Musterdatenbank dementsprechend groß wird.

Klassische, sequenzielle Algorithmen benötigen mit jedem Datensatz mehr Rechenzeit, um ein präsentiertes Muster zuzuordnen, was Echtzeit-Anwendungen nur eingeschränkt erlaubt und oft nur mittels leistungsfähiger und aufwändiger Prozessoren möglich macht.

Die im hier beschriebenen Beispiel genutzte CogniMem-Technologie erlaubt die Klassifizierung von Eingangsmustern in konstanter Rechenzeit (ca. 10 $\mu$s pro Muster), unabhängig von der Datenbankgröße (Anzahl der belegten Neuronen) und zu einem günstigen Preis (ca. 60 Euro pro Chip).

Wird dem neuronalen Netzwerk ein nach obiger Methode codiertes Zernike-Polynom vorgestellt, antwortet es nach einer Erkennungszeit automatisch mit der Kategorie (Datensatznummer) des zuvor angelernten Polynoms, das am besten dazu passt. Dies funktioniert auch, wenn dem Netzwerk abgewandelte, d.h. z.B. bekannte Polynome mit einer nicht in der Datenbank vorkommenden Amplitude, präsentiert werden. Die Erfindung nutzt diese Fähigkeit aus, unbekannte Eingangspolynome auf ein bekanntes Muster verallgemeinern zu können. Dies ist wesentlich, um eine gemessene Phasenverteilung in ihre entsprechende Linearkombination von Zernike-Polynomen zu zerlegen. Dies geschieht basierend auf einer zuvor angelernten Datenbank so gut wie möglich, d.h. mit dem geringsten Fehler.

Hierzu kommt ein sehr einfacher, iterativer Algorithmus zum Einsatz, der vergleichsweise nur sehr wenig Rechenzeit benötigt.

Bevorzugt wird für die voranstehend erläuterte Verfahrensvariante ein danach (siehe Gleichung (3,) (3.1), (3.2)) erstellter Eingangsvektor ermittelt, der eine Phasenverteilung beschreibt, die sich aus einer beliebigen Anzahl von Linearkombinationen der zuvor dem Netzwerk angelernten Zernike-Polynome zusammensetzt. Die folgende Gleichung beschreibt dies anhand eines Beispiels:

$$V_{PI} = \psi \, (\varphi_{P1} = 1 \cdot Z_4^0 - 1{,}2 \cdot Z_3^3 + 1{,}6 \cdot Z_{-2}^2 \,)$$
$$= (VP1_{X1} \quad VP1_{Y2} \quad \ldots \quad \ldots \quad VP1_{Xn} \quad VP1_{Yn}) \tag{7}$$

In Gleichung (5) ist $V_{P1}$ der resultierende Eingangsvektor für die beispielhafte Phasenverteilung $\varphi_1$, die die Elemente $VP1_{X1}$ bis $VP1_{Yn}$ umfassen kann.

Dieser Vektor wird nun einem neuronalen Netzwerk, z.B einem CogniMem-Chip, zur Erkennung präsentiert, der zuvor entsprechend Figur 4 angelernt wurde. Nach dem Ablauf eines Zeitraumes, der für die Erkennung benötigt wurde, liefert das neuronale Netzwerk das Muster zurück, das am besten zum präsentierten Vektor passt (Gleichung (8)):

$$\varphi_{R1} = \psi'(VR1_{X1} \quad VR1_{Y2} \quad \ldots \quad \ldots \quad VR1_{Xn} \quad VR1_{Yn}) = 1 \cdot Z_4^0 \tag{8}$$

Im Beispielpolynom $\varphi_{P1}$ stellt also $\varphi_{R1}$ den dominantesten Beitrag dar. Da $\varphi_{P1}$ eine Linearkombination aus den dem neuronalen Netzwerk bekannten Polynomen ist, wird die Differenz von $\varphi_{P1}$ und $\varphi_{R1}$ ebenfalls wieder eine Linearkombination bekannter Polynome sein. Diese Differenz wird in einen Eingangsvektor umgewandelt. Dieser wird in einem zweiten Erkennungsschritt erneut ausgewertet, und kann also beispielsweise hierzu dem CogniMem-Chip zur Auswertung übergeben (Gleichung (9)) werden.

$$V_{P2} = \psi \, (\varphi_{P2} = \varphi_{P1} - \varphi_{R1} = - 1{,}2 \cdot Z_3^3 + 1{,}6 \cdot Z_{-2}^2 \,)$$
$$= (VP2_{X1} \quad VP2_{Y2} \quad \ldots \quad \ldots \quad VP2_{Xn} \quad VP2_{Yn}) \tag{9}$$

Wie bereits beim vorherigen Erkennungsschritt (Gleichung (8)), wird das neuronale Netzwerk wiederum das dominanteste, bekannte Zenike-Polynom als in dem Vektor $V_{P2}$ enthaltenen Beitrag wieder finden (Gleichung (10)):

$$\varphi_{R2} = \psi'(VR2_{X1} \quad VR2_{Y2} \quad \ldots \quad \ldots \quad VR2_{Xn} \quad VR2_{Yn}) = 1{,}6 \cdot Z_{-2}^2 \tag{10}$$

Nun wird analog zu Gleichung (9) wiederum die Differenz zwischen dem präsentierten Polynom $\varphi_{P2}$ und $\varphi_{R2}$ gebildet und erneut dem neuronalen Netzwerk präsentiert (Gleichung (11)):

$$V_{P3} = \psi \, (\varphi_{P3} = \varphi_{P2} - \varphi_{R2} = - 1{,}2 \cdot Z_3^3 \,)$$
$$= (VP3_{X1} \quad VP3_{Y2} \quad \ldots \quad \ldots \quad VP3_{Xn} \quad VP3_{Yn}) \tag{11}$$

Der neue Eingangsvektor $V_{P3}$ wird erneut dem Netzwerk zur Erkennung übergeben und das Ergebnis (Gleichung (12)) ist eindeutig:

$$\varphi_{R3} = \psi'(VR3_{X1} \quad VR3_{Y2} \quad \ldots \quad \ldots \quad VR3_{Xn} \quad VR3_{Yn}) = - 1{,}2 \cdot Z_{-3}^3 \tag{12}$$

Die beschriebenen Schritte des erfindungsgemäßen Verfahrens a) Erkennung des dominanten Musters, b) Differenzbildung, und c) Präsentation des neuen Musters, werden so lange wiederholt, bis für das präsentierte Differenzmuster "0" erreicht wird (Gleichungen (13.1), (13.2)) oder ein anderes definierbares Abbruchkriterium (z.B. maximale Anzahl an Summanden der Linearkombination) erreicht wird.

$$\varphi_{Pk} = \varphi_{P(k-1)} - \varphi_{P(k-1)} = 0 \tag{13.1}$$

$$\varphi_{Rk} = 0 \tag{13.2}$$

In unserem Beispiel ist die Erkennung nach dem dritten Schritt abgeschlossen. Der vierte, dem Netzwerk präsentierte Vektor $V_{P4}$ wird als "0" erkannt werden (Gleichung (14)):

$$V_{P4} = \psi \ (\varphi_{P4} = \varphi_{P3} - \varphi_{R3} = 0)$$
$$= (VP4_{X1} \quad VP4_{Y2} \quad \ldots \quad \ldots \quad VP4_{Xn} \quad VP4_{Yn}) \tag{14}$$

Die Summe der erkannten Einzel-Polynome $\varphi_{R1} \ldots \varphi_{Rn}$ liefert dann die ursprüngliche Linearkombination $\varphi_{P1}$ (Gleichung (15)):

$$\varphi_{R(Total)} = \sum_{i-1}^{n} \varphi_{Ri} = \varphi_{P1} \tag{15}$$

Das neuronale Netzwerk ist also aufgrund seiner Fähigkeit zur Verallgemeinerung dazu in der Lage, eine gegebene Funktion in Linearkombinationen von bekannten linear unabhängigen Funktionen, z.B. Zernike-Polynomen, die sich in einem gültigen Eingangsvektor darstellen lassen, in wenigen Schritten zu zerlegen. Figur 6 illustriert die im Beispiel durchgeführten Operationen noch einmal grafisch.

Sollte einer der beschriebenen Erkennungsschritte tatsächlich einmal nicht sofort den dominantesten Polynom-Anteil (oder nicht in der richtigen Amplitude) zurückliefern, stellt dies keinen Mangel dar. Da der Algorithmus stets versucht, die Differenz der Phasenverteilung bestmöglich an "0" anzunähern, wird eine eventuelle Abweichung automatisch in einem der nachfolgenden Erkennungsschritte korrigiert.

Im Beispiel wurde dem Netzwerk eine Kombination präsentiert, die aus bekannten Komponenten besteht. Auf die Frage, was passiert, wenn eine oder mehrere Komponenten der Linearkombination dem Netzwerk nicht bekannt sind, wird dieses ebenfalls dank seiner Fähigkeit zur Verallgemeinerung antworten: Es wird dann die nächst beste bekannte Funktion auswählen. Zwar wird die Fehlerrechnung aus Gleichung (15) dann einen entsprechenden Fehler aufweisen. Dieser wird aber vom neuronalen Netzwerk automatisch so weit wie möglich minimiert.

Dieses Verhalten des neuronalen Netzwerks kann sogar für seine Anwendung ausgenutzt werden. So stellen z.B. bestimmte Zernike-Polynome spezielle optische Eigenschaften dar, z.B. Tilt, Focus oder sphärische Aberrationen. Zur Reduktion der Datenbankgröße und damit auch der Anzahl der nötigen Erkennungsschritte, kann man dem SHS einen Raumfilter, z.B. eine Fourieroptische Teleskopanordnung, vorschalten. Dann werden nur noch die niedrigen Polynomordnungen durchgelassen und vom SHS in Daten umgewandelt. Wird das neuronale Netzwerk also "nur" auf eine Datenbank aus diesen bestimmten Zernike-Polynomen mit einer physikalischen Relevanz spezieller Aberrationen angelernt, so wird das System versuchen, die präsentierten Messungen damit bestmöglich darzustellen. Das so gewonnene Ergebnis lässt sich dann als fehlerminimierte Messung der zuvor durch das Anlernen definierten optischen Eigenschaften interpretieren.

Die Erkennungszeit für eine komplette Linearkombination ist abhängig von der Anzahl von beteiligten Komponenten die dem neuronalen Netzwerk bekannt sind. Für das obige Beispiel beträgt die reine Erkennungszeit bei Verwendung eines CogniMem-Chips ca. 40 µs, da vier Erkennungsschritte nötig waren. Zu dieser Dauer addiert sich natürlich noch die Rechenzeit für die Bildung der Differenzen. Aufgrund der Linearität der Differentiation kann diese Differenzbildung direkt mit den Eingangsvektoren durchgeführt werden, ohne die realen Phasenfunktionen Rücktransformieren zu müssen. Dies lässt sich mit Ganzzahlarithmetik einfach lösen und kann gut auf Hardware (z.B. FPGA) implementiert werden.

Die vorgestellte Erfindung eignet sich insbesondere aufgrund der hohen Verarbeitungsgeschwindigkeit sehr gut zur Echtzeitmessung von Eigenschaften optischer Systeme im kHz Bereich und darüber hinaus.

Das vorgestellte Verfahren kann auch leicht auf die Dekomposition anderer Linearkombinationen eines Satzes bekannter linear unabhängiger Grundfunktionen übertragen werden. Das Verfahren ist damit generell auf jegliches Mess- oder Erkennungsproblem anwendbar, in dem eine Funktion einer oder mehrerer Variabler mathematisch in linear unabhängige Basisfunktionen zerlegt werden soll. Das Verfahren ist ebenfalls von der Technologie des neuronalen Netzwerks unabhängig, da es nur die Eigenschaften eines neuronalen Netzes nutzt, zu Erkennen, zu Klassifizieren und zu Verallgemeinern. Es hängt nicht davon ab, wie diese Eigenschaften in Hard- oder Software tatsächlich realisiert wurden.

**Patentansprüche**

1. Verfahren zur Darstellung einer Abtastfunktion mittels eines neuronalen Netzwerks, wobei das Verfahren

- eine wenigstens zweidimensionale Empfängeranordnung mit einer Anzahl von darauf verteilten, vorzugsweise optischen Sensoren bzw. Detektoren und
- eine wenigstens zweidimensionale Anordnung (10) von optischen Elementen, etwa Linsen, verwendet, die eine einfallende Wellenfront (100), welche als Abtastfunktion darstellbar ist, in Phaseninformationen enthaltende Bestandteile wandelt, mit folgenden Schritten:
- die Abtastfunktion wird dem neuronalen Netzwerk zur Verarbeitung übermittelt,
- eine Anzahl von Vergleichsfunktionen wird dem neuronalen Netzwerk zur Verfügung gestellt,

**dadurch gekennzeichnet,**

- **dass** durch Vergleich der Abtastfunktion mit mehreren der Vergleichsfunktionen eine primäre Vergleichsfunktion durch das neuronale Netzwerk selektiert wird, wobei die primären Vergleichsfunktionen einer vollständigen Funktionenbasis entstammen bzw. wenigstens eine Untermenge der Funktionen einer derartigen Funktionenbasis bilden,
- **dass** die selektierte primäre Vergleichsfunktion als dominante Repräsentationsfunktion bestimmt wird,
- **dass** die Repräsentationsfunktion von der Abtastfunktion abgezogen wird und die Differenz wie die ursprüngliche Abtastfunktion prozessiert wird,
- **dass** eine Differenzfunktion gebildet wird, bis der vorgebbare Wert unterschritten worden ist oder ein anderes Abbruchkriterium erreicht worden ist,
- **dass** die ermittelten Repräsentationsfunktionen in ihrer Gesamtheit die Abtastfunktion darstellen.

2. Verfahren nach Anspruch 1, **dadurch gekennzeichnet, dass** das neuronale Netzwerk über eine möglichst eng aufeinanderfolgende Schrittweite für die Diskretisierung der Amplitude der Vergleichsfunktion und/oder

   einen breiten Bereich für die Vergleichsfunktionen
   und/oder eine möglichst große Anzahl von gelernten Vergleichsfunktionen verfügt.

3. Verfahren nach einem der voranstehenden Ansprüche, **dadurch gekennzeichnet, dass** die Abtastfunktion einem Eingangsmuster entspricht, das dem neuronalen Netzwerk zur Erkennung der darin enthaltenen Repräsentationsfunktionen übermittelt wird.

4. Verfahren nach einem der voranstehenden Ansprüche, **dadurch gekennzeichnet, dass** ein Sender eingesetzt wird und die Respons der Strahlung des Senders wenigstens zwei-dimensional aufgelöst wird, wobei zwei-dimensional verteilte Sensoren oder Detektoren die Respons-Strahlung in ein Eingangsmuster wandeln.

5. Verfahren nach einem der vorstehenden Ansprüche, **dadurch gekennzeichnet, dass** eine optische Vorfilterung nach Raumfrequenzen durchgeführt wird, so dass das Eingangsmuster vereinfacht wird.

6. Verfahren nach einem der voranstehenden Ansprüche, **dadurch gekennzeichnet, dass** ein die Abtastfunktion darstellendes Eingangsmuster als ein Eingangsvektor dem neuronalen Netzwerk zugeführt wird und die Vergleichsfunktionen dem Netzwerk in der Form von Vektoren zugänglich sind, um die Darstellung durchzuführen.

7. Verfahren nach einem der voranstehenden Ansprüche, **dadurch gekennzeichnet, dass** die Differenz in einen Eingangsvektor gewandelt wird und dem neuronalen Netzwerk zur weiteren Darstellung übermittelt wird.

8. Vorrichtung zur Darstellung einer Abtastfunktion mittels eines neuronalen Netzwerks mit folgenden Merkmalen:

   - einer wenigstens zweidimensionalen Empfängeranordnung mit einer Anzahl von darauf verteilten, vorzugsweise optischen Sensoren bzw. Detektoren;
   - einer wenigstens zweidimensionalen Anordnung (10) von optischen Elementen, etwa Linsen, die eine einfallende Wellenfront (100), welche als Abtastfunktion darstellbar ist, in Phaseninformationen enthaltende Bestandteile wandelt;
   - einem neuronalen Netzwerk, in das die Bestandteile, die von der Empfängeranordnung kommen, eingegeben werden;
   - wobei das neuronale Netzwerk auf eine Anzahl von Vergleichsfunktionen zurückgreift, um die Bestandteile der Abtastfunktion mit wenigstens einer der Vergleichsfunktionen zu beschreiben;

**dadurch gekennzeichnet,**

**dass** die Vorrichtung zur Ausführung des Verfahrens nach einem der Ansprüche 1 bis 7 ausgebildet ist.

9. Vorrichtung nach Anspruch 8, **dadurch gekennzeichnet, dass** eine Einrichtung zur optischen Vorfilterung vorgesehen ist, die die Abtastfunktionen einer Vorfilterung nach Raumfrequenzen unterzieht.

10. Vorrichtung nach Anspruch 8 oder 9, **dadurch gekennzeichnet, dass** wenigstens ein Sender zur Erzeugung einer bevorzugt optischen Strahlung vorgesehen ist.

11. Vorrichtung nach einem der voranstehenden Vorrichtungsansprüche, **dadurch gekennzeichnet, dass** die Anordnung einen Shack-Hartmann-Sensor oder einen Vierwellenlateral-Hearing Interferometriesensor umfasst.

12. Vorrichtung nach einem der voranstehenden Vorrichtungsansprüche, **dadurch gekennzeichnet, dass** die wenigstens zweidimensionale Empfängeranordnung und die wenigstens zweidimensionale Anordnung von optischen Elementen, die einen Phasenfrontdetektor bilden, und das neuronale Netzwerk eine Funktionseinheit bilden, die als Messdaten nur die Koeffizienten der Basisentwicklung ausgeben.

## Claims

1. Process for the representation of a scanning function by means of a neuronal network, consisting of:

   - an at least two-dimensional array of receivers with a plurality of sensors and/or detectors distributed thereon.
   - an at least two-dimensional array of optical elements (10), such as lenses, which converts the incident wave front, which can be represented as scanning function, into component parts which contain phase information, with the following steps:
   - the scanning function is transmitted to the neuronal network for processing.
   - a number of comparison functions are made available to the neuronal network.

   **characterized in**

   - **that** a primary comparison function is selected by the neuronal network through comparison of the scanning function with several of the comparison functions, wherein the primary comparison functions stem from a complete function base or form at least a subgroup of this function base,
   - wherein the selected primary comparison function is determined as dominant representation function,
   - wherein the representation function is subtracted from the scanning function and the difference is processed like the original scanning function,
   - wherein a difference function is formed until a result falls below the predeterminable value or until another end criterion is reached,
   - wherein the determined representation functions as a whole form the scanning function.

2. Process according to claim 1, wherein the neuronal network has a successive step width that is as narrow as possible for the discretization of the amplitude of the comparison function, and/or a broad range for the comparison functions and/or the largest possible number of learnt representation functions.

3. Process according to one of the upper claims, wherein the scanning function corresponds to an input vector, which is transmitted to the neuronal network for further analysis of the representation functions contained within the neuronal network.

4. Process according to one of the upper claims, wherein a transmitter is used and a response to the transmitter radiation disperses at least two-dimensionally, whereby two-dimensionally distributed sensors or detectors convert the response radiation into an input pattern.

5. Process according to one of the upper claims, wherein optical pre-filtering according to spatial frequencies is performed, so that the input pattern is simplified.

6. Process according to one of the upper claims, wherein a sample pattern which represents the scanning function is brought to the neuronal network in the form of an input vector, and the comparison functions are accessible to the network in the form of vectors in order to perform the representation.

7. Process according to one of the upper claims, wherein the difference is converted into an input vector and transmitted to the neuronal network for further representation.

8. Device for the representation of a scanning function by means of a neuronal network, with the following characteristics:

   - an at least two-dimensional array of receivers wherein a plurality of sensors and/or detectors and preferably optical ones, are distributed thereon.
   - An at least two-dimensional array of optical elements (10), such as lenses, which convert the incident wave front (100), representable as scanning function, into component parts which contain phase information;
   - A neuronal network in which component parts coming from the receiver array are entered;
   - Whereby the neuronal network has access to a plurality of comparison functions, in order to describe component parts of the scanning function with at least one of the comparison functions;

   **characterized in**
   the device for the processing being configured according to one of the claims 1 to 7.

9. Device according to claim 8, wherein an optical pre-filtering facility is provided, which subjects the scanning function to pre-filtering according to spatial frequencies.

10. Device according to claim 8 or 9, wherein at least one transmitter is provided for the generation of a preferably optical radiation.

11. Device according to one of the upper claims wherein an arrangement of the at least two-dimensional receiver array includes a Shack-Hartmann sensor or a quadri-wave lateral shearing interferometry sensor.

12. Device according to one of the upper claims, wherein the at least two-dimensional receiver array and the at least two-dimensional array of optical elements, which form a phase front detector, and the neuronal network form a single functional unit, which, as measuring data, outputs only the coefficients of the base development.

**Revendications**

1. L'invention décrit un procédé pour représenter une fonction d'échantillonnage par un réseau neuronal, ce procédé consistant en:

   - au minimum une configuration bidimensionnelle de récepteurs avec un nombre de capteurs ou détecteurs, de préférence optiques, distribués sur la surface ;
   - l'utilisation d'au minimum une configuration bidimensionnelle (10) d'éléments optiques, par exemple de lentilles, qui convertit un front d'onde incident (100), qui peut être représenté éventuellement par une fonction d'échantillonnage, en éléments contenants les informations des phases, en passant par les étapes suivantes :
   - la fonction d'échantillonnage est transmise au réseau neuronal pour traitement,
   - un nombre de fonctions de comparaison est mis à la disposition du réseau neuronal, étant **caractérisé en ce que**
   - par comparaison de la fonction d'échantillonnage avec plusieurs fonctions de comparaison, le réseau neuronal sélectionne une fonction de comparaison primaire, la fonction de comparaison primaire étant issue d'une base de fonctions complète ou issue au moins d'un sous-groupe des fonctions d'une telle base de fonctions.
   - la fonction de comparaison primaire sélectionnée est définie comme une fonction de représentation dominante,
   - la fonction de représentation est soustraite de la fonction d'échantillonnage et la différence est traitée comme la fonction d'échantillonnage d'origine,
   - une fonction différentielle est formée jusqu'à ce que sa valeur passe en dessous du seuil prédéterminé ou qu'un autre critère d'interruption soit rempli.
   - les fonctions représentatives déterminées représentent dans l'ensemble la fonction d'échantillonnage et d'analyse.

2. Procédé selon revendication 1, **caractérisé en ce que** le réseau neuronal dispose d'incréments les plus rapprochés possible pour discrétiser l'amplitude de la fonction de comparaison et/ou d'une large plage pour les fonctions de comparaison et/ou le plus grand nombre possible de fonctions de comparaison apprises.

3. Procédé selon une des revendications précédentes, **caractérisé en ce que** la fonction d'échantillonnage correspond à un échantillon d'entrée, qui est transmis au réseau neuronal pour la reconnaissance des fonctions de représentation qui y sont contenues.

4. Procédé selon une des revendications précédentes, **caractérisé en ce qu'**un émetteur est utilisé et que la réponse du rayonnement de l'émetteur est résolue sous forme bidimensionnelle, les capteurs et détecteurs distribués à minima de façon bidimensionnelle transformant le rayonnement en un échantillon d'entrée.

5. Procédé selon une des revendications précédentes, caractérisé en ce quun pré filtrage optique des fréquences spatiales est réalisé pour simplifier l'analyse d'entrée.

6. Procédé selon une des revendications précédentes, **caractérisé en ce qu'**un échantillon d'entrée représentant la fonction d'échantillonnage est transmis au réseau neuronal comme vecteur d'entrée et que les fonctions de comparaison sont accessibles au réseau neuronal sous forme de vecteurs pour la représentation.

7. Procédé selon une des revendications précédentes, **caractérisé en ce que** la différence se transforme en vecteur d'entrée et qu'elle est transférée au réseau neuronal pour la représentation suivante.

8. Dispositif pour la présentation d'une fonction d'échantillonnage par un réseau neuronal avec les caractéristiques suivantes:

- à minima une configuration bidimensionnelle de récepteurs avec un nombre de capteurs ou détecteurs, de préférence optiques, sur la surface de celui-ci;
- d'une configuration au minimum bidimensionnelle (10) d'éléments optiques, par exemple des lentilles, qui convertit un front d'onde incident (100) qui peut être représenté comme fonction de balayage (échantillonnage) en composants contenant des informations de phase;
- un réseau neuronal dans lequel sont introduits les composants provenant de l'ensemble récepteur
- le réseau neuronal se basant sur un nombre de fonctions de comparaison pour décrire les éléments constitutifs de la fonction d'échantillonnage avec au moins une fonction de comparaison;

**Caractérisé en ce que**
le dispositif pour l'exécution du procédé est fait selon une des revendications 1 à 7.

9. Dispositif selon la revendication 8, **caractérisé en ce qu'**il est prévu un dispositif de pré-filtrage optique qui soumet les fonctions de balayage à un pré-filtrage selon les fréquences spatiales.

10. Dispositif selon la revendication 8 ou 9, **caractérisé en ce qu'**au minimum un émetteur est prévu pour la production d'un rayonnement de préférence optique.

11. Dispositif selon l'une quelconque des revendications de dispositif précédentes, **caractérisé en ce que** le dispositif comprend un capteur Shack-Hartmann ou un capteur interférométrique à résonance latérale à quatre ondes.

12. Dispositif selon l'une quelconque des revendications précédentes, **caractérisé en ce que** le réseau de récepteurs au moins bidimensionnel et le réseau au moins bidimensionnel d'éléments optiques formant un détecteur de front de phase, et le réseau neuronal formant une unité fonctionnelle qui ne fournit comme données de mesure que les coefficients de développement de fonctions de base ainsi déterminées.

# Figur 1

# Figur 2

# Figur 3

# Figur 4

# Figur 5

$Z_4^{+4}$    $Z_4^{+4}$    $Z_4^{+4}$    $Z_4^{+4}$    $Z_4^{+4}$

$\cdots$    $\alpha = -0,2\lambda$    $-0,1\lambda$    $0,1\lambda$    $0,2\lambda$    $0,3\lambda$ $\cdots$

# Figur 6

**Präsentiertes Muster**      **Erkanntes Muster**

①   $\varphi_{P1} = 1 \cdot Z_4^0 - 1,2 \cdot Z_3^3 + 1,6 \cdot Z_{-2}^2$  →  $\varphi_{R1} = 1 \cdot Z_4^0$

②   $\varphi_{P2} = \varphi_{P1} - \varphi_{R1} = -1,2 \cdot Z_3^3 + 1,6 \cdot Z_{-2}^2$  →  $\varphi_{R2} = 1,6 \cdot Z_{-2}^2$

③   $\varphi_{P3} = \varphi_{P2} - \varphi_{R2} = -1,2 \cdot Z_3^3$  →  $\varphi_{R3} = -1,2 \cdot Z_3^3$

④   $\varphi_{P4} = \varphi_{P3} - \varphi_{R3} = 0$  →  Erkennung abgeschlossen

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **GUO, H. et al.** Wavefront reconstruction with artificial neural networks. *OPTICS EXPRESS,* 10. Juni 2006, vol. 14 (14), 6456-6462